# DEMANDE DE BREVET EUROPEEN

(11) **EP 4 159 198 A1**
(43) Date de publication de la demande: **05.04.2023**
(21) Numéro de dépôt: 22199431.2
(22) Date de dépôt: 03.10.2022
(51) Int. Cl.: A61K 9/14, A61K 9/16, A61K 9/20, A61K 8/97, A61K 36/53, A61K 8/02, A61K 8/73, A23F 3/34, A23L 33/105, A61K 8/72, A61K 8/9789, A61Q 19/00, A23P 10/20, A23P 10/40, A23L 29/30

(54) **PROCÉDÉ DE FABRICATION D'UNE POUDRE COMPRENANT UN SUPPORT BIOLOGIQUE SUR LEQUEL EST DÉPOSÉ UN EXTRAIT BIOLOGIQUE**

(30) Priorité: 04.10.2021 FR 2110475
(71) Demandeur: Larena, 49270 Oree D'Anjou (FR)
(72) Inventeur: BARDOT, Valérie, 03140 TARGET (FR); DUBOURDEAUX, Alexandre, 03800 GANNAT (FR)
(74) Mandataire: Jacobacci Coralis Harle

(57) **Abrégé**

La présente invention concerne un procédé de fabrication d'une poudre imprégnée comprenant un support biologique sur lequel est déposé un extrait biologique.

Ce procédé de fabrication comprend une étape d'imprégnation d'un extrait biologique concentré sur un support biologique, puis une étape de séchage dudit support biologique imprégné par ledit extrait biologique concentré, pour obtenir ladite poudre imprégnée.

Le procédé de fabrication comprend une étape de micronisation pour obtenir une poudre imprégnée présentant, après ladite étape de séchage, les caractéristiques granulométriques suivantes : - une taille inférieure, ou égale, à 50 µm représentant au maximum 25%, de préférence de 10 à 25%, - une taille inférieure, ou égale, à 100 µm représentant au maximum 50%, de préférence de 25 à 50%, et - une taille inférieure, ou égale, à 500µm représentant au minimum 90%, de préférence de 95 à 100%.

## Description

### Domaine technique de l'invention

La présente invention concerne le domaine technique des poudres comprenant un support biologique sur lequel est déposé un extrait biologique.

Elle concerne en particulier le procédé de fabrication d'une telle poudre, ainsi que la poudre en tant que telle.

### Etat de la technique

Certaines formes galéniques ont l'intérêt de présenter des caractéristiques intéressantes sur le plan de l'activité et de l'assimilation.

En ce sens, il existe par exemple des poudres dites « imprégnées », comprenant un support biologique sur lequel est déposé un extrait biologique.

C'est par exemple le cas d'extraits végétaux qui sont fixés sur un support cellulosique. Une telle forme galénique, avec son procédé de fabrication, est décrite par exemple dans le document EP-2 080 436.

Une telle approche est intéressante pour augmenter la disponibilité de principes actifs, sans toutefois affecter le « totum » de la matière biologique.

Mais, en pratique, les poudres imprégnées de l'art antérieur ne présentent pas toujours un profil de libération adapté à l'application envisagée.

### Présentation de l'invention

Afin de remédier à l'inconvénient précité de l'état de la technique, la présente invention propose un procédé de fabrication d'une poudre imprégnée comprenant un support biologique sur lequel est déposé un extrait biologique.

Le procédé de fabrication selon l'invention comprend :
- une étape de fourniture d'un extrait biologique concentré,
ledit extrait biologique concentré étant issu d'une opération d'extraction à partir d'une matière biologique suivi d'une opération de concentration dudit extrait biologique,
- une étape d'imprégnation dudit extrait biologique concentré sur ledit support biologique, par exemple une étape de pulvérisation, et
- une étape de séchage dudit support biologique imprégné par ledit extrait biologique concentré, pour obtenir ladite poudre imprégnée.

Et, selon l'invention, ledit procédé de fabrication comprend une étape de micronisation dudit support biologique et/ou de ladite poudre imprégnée dont les paramètres sont ajustés pour obtenir une poudre imprégnée présentant, après ladite étape de séchage, les caractéristiques granulométriques suivantes, exprimées en pourcentage massique du poids total de ladite poudre imprégnée :
- une taille inférieure, ou égale, à 50 µm représentant au maximum 25%, de préférence de 10 à 25%,
- une taille inférieure, ou égale, à 100 µm représentant au maximum 50%, de préférence de 25 à 50%, et
- une taille inférieure, ou égale, à 500µm représentant au minimum 90%, de préférence de 95 à 100%.

De manière surprenante et inattendue, une telle poudre imprégnée, selon l'invention, présente un profil de libération différent de celui des poudres imprégnées de l'art antérieur, avantageusement une libération modifiée d'une substance active, de préférence une libération lente et progressive (avantageusement pendant au moins 4 heures).

Une telle poudre imprégnée selon l'invention peut ainsi être envisagée dans de nouvelle application.

D'autres caractéristiques non limitatives et avantageuses du procédé conforme à l'invention, prises individuellement ou selon toutes les combinaisons techniquement possibles, sont les suivantes :
- ladite poudre imprégnée présente : une masse volumique apparente, ou plus précisément une masse volumique apparente sans tassement, allant de 0,3 à 0,8 g/cm³, et une masse volumique tassée, ou masse volumique apparente après tassement, allant de 0,4 à 0,9 g/cm³ ;
- le support biologique est un support végétal et l'extrait biologique est un extrait végétal ;
- ladite étape de micronisation est mise en œuvre sur le support biologique préalablement à ladite étape d'imprégnation, et/ou entre ladite étape d'imprégnation et ladite étape de séchage, et/ou postérieurement à ladite étape de séchage ;
- ledit extrait biologique concentré est issu d'une opération d'extraction à partir d'une matière végétale fraîche ou sèche, laquelle opération d'extraction consiste par exemple en une opération d'extraction aqueuse, éventuellement par un solvant consistant en un mélange hydro-alcoolique, avantageusement hydro-éthanolique, et encore plus avantageusement un mélange 50% / 50% en volume ;
- ledit extrait biologique concentré est issu d'une opération d'extraction réalisée sous chauffage, avantageusement sous hyperfréquence, et sous pression réduite ou est réalisée sous gaz inerte, avantageusement sous azote ;
- ledit extrait biologique concentré est issu d'une opération d'extraction mise à œuvre à une température inférieure ou égale à 95°C à 150 mbar, et encore plus avantageusement à une température de 55°C sous pression réduite de 150 mbar et en ce qu'elle a une durée inférieure à 1 heure, encore plus avantageusement de l'ordre de 15 minutes ;
- ledit extrait biologique concentré est issu d'une opération d'extraction mise à œuvre plusieurs fois, avantageusement trois fois, et de préférence, entre chaque répétition, de la matière végétale est ajouté à l'extrait et/ou la température d'extraction est augmentée.

La présente invention concerne encore la poudre imprégnée issue d'un procédé de fabrication selon l'invention.

Cette poudre imprégnée comprend un support biologique sous forme d'une poudre sur lequel est déposé un extrait biologique concentré.

La poudre imprégnée présente les caractéristiques granulométriques suivantes :
- une taille inférieure, ou égale, à 50 µm représentant au maximum 25%, de préférence de 10 à 25%,
- une taille inférieure, ou égale, à 100 µm représentant au maximum 50%, de préférence de 25 à 50%, et
- une taille inférieure, ou égale, à 500µm représentant au minimum 90%, de préférence de 95 à 100%,
laquelle poudre imprégnée présente avantageusement :
- une masse volumique apparente, ou plus précisément une masse volumique apparente sans tassement, allant de 0,3 à 0,8 g/cm³, et
- une masse volumique tassée, ou masse volumique apparente après tassement, allant de 0,4 à 0,9 g/cm³.

La présente invention concerne encore une composition, avantageusement sous forme d'un comprimé, comprenant une poudre imprégnée selon l'invention mélangée avec au moins un principe actif.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

De plus, diverses autres caractéristiques de l'invention ressortent de la description annexée, en lien avec les figures dans lesquelles :

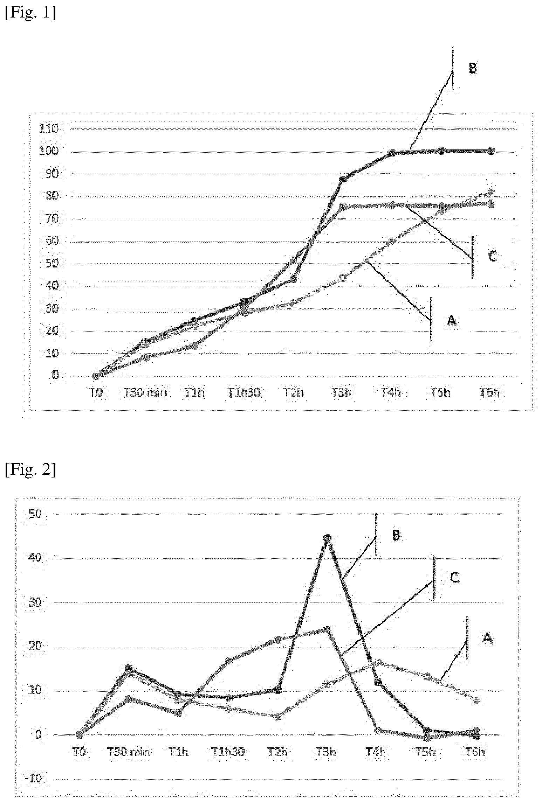

[Fig. 1] représente les profils de dissolution en cumulé pour trois formulations : formulation à base d'une poudre imprégnée selon l'invention (A), formulation à base d'une poudre imprégnée selon l'art antérieur (B), extrait sec (C), en pourcentage cumulé en fonction du temps en heure ;
[Fig. 2] représente les profils de dissolution en instantané pour trois formulations : formulation à base d'une poudre imprégnée selon l'invention (A), formulation à base d'une poudre imprégnée selon l'art antérieur (B), extrait sec (C), en pourcentage instantané en fonction du temps en heure.

La présente invention concerne ainsi un procédé de fabrication d'une poudre imprégnée, y compris la poudre imprégnée en tant que telle.

De manière générale et tel que développé par la suite, la poudre imprégnée comprend un support biologique sur lequel est déposé (dit encore « fixé ») un extrait biologique concentré.

De telles poudres imprégnées sont avantageusement adaptées à une administration par voie orale chez un sujet humain ou chez un sujet animal.

La poudre imprégnée est notamment utilisable dans des compositions alimentaires (notamment un complément alimentaire), cosmétiques, pharmaceutiques ou en nutrition animale.

Pour l'obtention d'une telle poudre imprégnée, le procédé de fabrication selon l'invention comprend :
- une étape de fourniture d'un extrait biologique concentré,
- une étape d'imprégnation dudit extrait biologique concentré sur un support biologique, et
- une étape de séchage dudit support biologique imprégné par ledit extrait biologique concentré, pour obtenir ladite poudre imprégnée.

Selon l'invention, ce procédé de fabrication selon l'invention comprend encore une étape de micronisation dudit support biologique et/ou de ladite poudre imprégnée, dans laquelle les paramètres de ladite étape de micronisation sont ajustés pour obtenir une poudre imprégnée présentant, après ladite étape de séchage, des caractéristiques granulométriques particulières détaillées ci-après.

### Extrait biologique concentré et étape de fourniture

Par « extrait biologique concentré », on entend avantageusement une fraction obtenue par extraction à partir d'une matière biologique puis par concentration de ladite fraction extraite.

La concentration des composés d'intérêt qui sont présents dans l'extrait biologique concentré est supérieure à la concentration des composés d'intérêt qui sont présents dans la matière biologique d'origine.

A cet effet, l'extrait biologique concentré est issu d'une opération d'extraction à partir d'une matière biologique suivie d'une opération de concentration dudit extrait biologique.

De telles opérations d'extraction / concentration sont par exemple décrites dans le document EP-2 080 436.

Une telle opération d'extraction comprend avantageusement les étapes suivantes :
- une étape de contact entre la matière biologique d'intérêt et un solvant,
- au moins une étape d'extraction, proprement dite, d'au moins une partie des principes actifs présents dans ladite matière biologique d'intérêt (par migration dans le solvant).

L'opération de concentration consiste alors avantageusement à concentrer les principes actifs par élimination d'au moins une partie du solvant d'extraction.

La concentration est avantageusement d'un facteur de concentration d'au moins 5, de préférence d'un facteur de concentration de 8 à 12.

Selon une forme de réalisation préféré, l'extrait biologique concentré consiste en un extrait végétal concentré. L'extrait biologique concentré est issu d'une opération d'extraction à partir d'une matière végétale, suivie d'une opération de concentration dudit extrait biologique.

Dans ce cadre, la matière végétale consiste avantageusement en une matière végétale fraîche ou sèche, avantageusement sur une partie de plante ou une plante entière.

L'opération d'extraction comprend ainsi avantageusement une mise en contact de la matière végétale avec un solvant, dans lequel va avoir lieu l'extraction des principes actifs.

De préférence encore, l'opération d'extraction consiste en une opération d'extraction aqueuse, éventuellement par un solvant consistant en un mélange hydroalcoolique, avantageusement hydro-éthanolique, et encore plus avantageusement un mélange 50% / 50% en volume.

De manière générale, une telle opération d'extraction est avantageusement réalisée sous chauffage, avantageusement sous hyperfréquence, et sous pression réduite ou est réalisée sous gaz inerte, avantageusement sous azote.

De manière avantageuse encore, cette extraction est réalisée sous hyperfréquence, ou tout autre moyen de chauffage conventionnel, et sous pression réduite. Ces deux conditions réunies permettent d'extraire efficacement les principes actifs de la plante et ainsi de respecter la notion d'intégralité, sans modification de leur intégrité.

De manière préférentielle, un générateur micro-onde GMP 60 K, équipant une enceinte d'extraction, délivre une puissance de 600 à 6000 W dans la bande de fréquence comprise entre 300 MHz et 300 GHz, avantageusement 2450 MHz.

L'utilisation des hyperfréquences comme moyen de chauffage, par rapport aux techniques conventionnelles, permet d'écourter la phase d'extraction car le chauffage de la masse végétale est très court.

La notion de pression réduite implique une mise sous vide, au moins partiel, ou sous gaz inerte. La valeur de la pression est alors comprise entre 50 et 300 mbar, avantageusement de l'ordre de 150 mbar. L'azote est avantageusement utilisé comme gaz inerte.

Sous l'effet conjugué du chauffage, avantageusement par hyperfréquence, et de la pression réduite, l'extraction se déroule jusqu'à épuisement de la matière végétale traitée.

De préférence encore, l'extrait biologique concentré est issu d'une opération d'extraction mise à œuvre à une température inférieure ou égale à 95°C à 150 mbar, et encore plus avantageusement à une température de 55°C sous pression réduite de 150 mbar.

L'opération d'extraction présente avantageusement une durée inférieure à 1 heure, encore plus avantageusement de l'ordre de 15 minutes.

Encore de manière générale, l'extrait biologique concentré est avantageusement issu d'une opération d'extraction mise à œuvre plusieurs fois, avantageusement trois fois.

De préférence, entre chaque répétition, de la matière végétale est ajouté à l'extrait et/ou la température d'extraction est augmentée.

De manière alternative, l'extrait biologique concentré pourrait consister en un extrait concentré en microorganismes d'intérêt et en milieu de fermentation.

### Support biologique et étape d'imprégnation

Le procédé de fabrication comprend ensuite une étape d'imprégnation de l'extrait biologique concentré sur le support biologique, avantageusement par une mise en contact de l'extrait biologique concentré et du support biologique.

Par exemple, cette étape d'imprégnation consiste en une étape de pulvérisation, de préférence une étape de pulvérisation de l'extrait biologique concentré sur le support biologique.

De préférence, le support biologique se présente sous la forme d'une poudre.

Selon une forme de réalisation préférée, le support biologique consiste en un support végétal.

Dans ce cadre, le support végétal consiste avantageusement en une matière végétale sèche, sous forme de poudre, avantageusement une partie de plante ou une plante entière.

De préférence, le support biologique est de même nature que la matière biologique utilisée pour l'obtention de l'extrait biologique concentré.

En ce sens, l'extrait biologique concentré et le support biologique sont avantageusement un extrait végétal concentré et un support végétal.

De manière générale, l'extrait biologique concentré peut comprendre un extrait concentré en microorganismes d'intérêt et en milieu de fermentation.

### Etape de séchage

L'étape de séchage correspond à une phase de séchage en présence du support biologique sur lequel a été déposé l'extrait biologique concentré au cours de l'opération d'imprégnation.

Cette étape de séchage a pour but essentiel d'entraîner l'évaporation du solvant apporté par l'extrait biologique concentré.

Cette étape de séchage est avantageusement mise en œuvre jusqu'au séchage complet de la poudre imprégnée.

De manière simultanée, ce séchage permet la fixation des principes actifs (apportés par l'extrait biologique concentré) sur le support biologique.

A l'issue du procédé, la poudre imprégnée se présente donc une forme sèche.

Une telle étape de séchage est par exemple décrite dans le document EP-2 080 436.

### Poudre imprégnée et étape de micronisation

Selon l'invention, le procédé de fabrication comprend encore l'étape de micronisation du support biologique et/ou de la poudre imprégnée, qui est ajustée pour obtenir une poudre imprégnée présentant, après l'étape de séchage, des caractéristiques granulométriques particulières.

En d'autres termes, l'étape de micronisation du support biologique est ajustée pour obtenir, à l'issue du procédé de fabrication, une poudre imprégnée présentant des caractéristiques granulométriques déterminées.

Par « micronisation », on entend avantageusement l'opération ayant pour objet la réduction du support biologique en particules ayant des dimensions de l'ordre du micromètre (ou micron).

Le paramétrage de l'étape de micronisation, classique en soi, est notamment fonction de la technologie utilisée (par exemple au moyen d'un microniseur ou d'un broyeur) et de l'ordre des étapes du procédé de fabrication.

Une telle étape de micronisation est par exemple décrite dans le document général suivant : Powder Technology Handbook, Fourth Edition, Higashitani et al.

De manière générale, les paramètres de l'étape de micronisation sont ajustés pour obtenir une poudre imprégnée présentant, après l'étape de séchage, des paramètres de distribution déterminés.

A cet égard, les caractéristiques granulométriques de la poudre imprégnée sont les suivantes :
- une taille inférieure, ou égale, à 50 µm représentant au maximum 25%, de préférence de 10 à 25%,
- une taille inférieure, ou égale, à 100 µm représentant au maximum 50%, de préférence de 25 à 50%, et
- une taille inférieure, ou égale, à 500µm représentant au minimum 90%, de préférence de 95 à 100%.

De manière classique en soi, les caractéristiques granulométriques sont avantageusement issues d'une technique granulométrique par tamisage.

Le pourcentage en masse en question représente avantageusement la partie de la poudre imprégnée qui reste sur le tamis, dit encore « refus » ou « refus par tamis ».

De telles valeurs peuvent être mesurées selon un protocole décrit par exemple dans le document suivant : Pharmacopée Européenne 9.0, « 2.9.38. Distribution granulométrique par tamisage analytique » ou Pharmacopée Européenne 9.0, « 2.9.35. Finesse des poudres ».

De préférence, une telle poudre imprégnée présente encore avantageusement :
- une masse volumique apparente, ou plus précisément une masse volumique apparente sans tassement, allant de 0,3 à 0,8 g/cm³, et
- une masse volumique tassée, ou masse volumique apparente après tassement, allant de 0,4 à 0,9 g/cm³.

La masse volumique apparente et la masse volumique tassée sont avantageusement obtenues par le procédé décrit dans le document Pharmacopée Européenne 9.7, « 2.9.34. Masse volumique vrac et masse volumique après tassement ».

De manière générale, selon l'invention, l'étape de micronisation est avantageusement mise en œuvre sur le support biologique :
- préalablement à l'étape d'imprégnation, de sorte à ajuster les caractéristiques granulométriques du support biologique avant l'étape d'imprégnation, et/ou
- entre l'étape d'imprégnation et l'étape de séchage, de sorte à ajuster les caractéristiques granulométriques du support biologique imprégné non-séché, et/ou
- postérieurement à l'étape de séchage, de sorte à ajuster les caractéristiques granulométriques du support biologique imprégné et séché.

### Poudre imprégnée

La présente invention concerne encore la poudre imprégnée issue du procédé de fabrication selon l'invention.

Cette poudre imprégnée comprend un support biologique sous forme d'une poudre sur lequel est déposé un extrait biologique concentré.

La poudre imprégnée présente les caractéristiques granulométriques suivantes :
- une taille inférieure, ou égale, à 50 µm représentant au maximum 25%, de préférence de 10 à 25%,
- une taille inférieure, ou égale, à 100 µm représentant au maximum 50%, de préférence de 25 à 50%, et
- une taille inférieure, ou égale, à 500µm représentant au minimum 90%, de préférence de 95 à 100%.

De préférence, la poudre imprégnée présente encore :
- une masse volumique apparente, ou plus précisément une masse volumique apparente sans tassement, allant de 0,3 à 0,8 g/cm³, et
- une masse volumique tassée, ou masse volumique apparente après tassement, allant de 0,4 à 0,9 g/cm³.

### Composition

La présente invention concerne également une composition, avantageusement sous forme d'un comprimé, comprenant un mélange entre :
- une poudre imprégnée selon l'invention, et
- au moins un principe actif.

La forme de réalisation « comprimé », avec son procédé de fabrication, est par exemple enseignée dans les documents Phi41, Pharmacotechnie industrielle, Edition IMT, 07/2016 ou Pharmacie galénique: Bonnes pratiques de fabrication des médicaments, Edition Elsevier Masson, 10e édition (19 octobre 2016).

Par « principes actifs », on englobe les probiotiques, bactéries, levures, plantes et extraits de plantes, vitamines, minéraux et oligoéléments, substances nutritionnelles, etc.

Plus généralement, le principe actif peut être choisi parmi un agent pharmaceutique, un agent vétérinaire, un complément nutritionnel, un agent agricole, un ingrédient cosmétique, un colorant et un aliment.

La composition selon l'invention peut encore contenir tout autre excipient.

La composition selon l'invention consiste avantageusement en une composition alimentaire (notamment un complément alimentaire), cosmétique ou pharmaceutique.

Bien entendu, diverses autres modifications peuvent être apportées à l'invention dans le cadre des revendications annexées.

La présente invention est encore illustrée au travers des exemples ci-dessous.

### Exemple

Trois lots de poudres imprégnés sont comparés : une poudre imprégnée selon l'invention, une poudre imprégnée selon l'état de la technique (grossière - conforme au document EP-2 080 436) et un extrait sec.

L'extrait sec est un extrait aqueux de mélisse qui a été séché par atomisation avec un support de maltodextrine.

Cet extrait sec présente la composition suivante composition :
- extrait natif de plante : 80 - 90%, et
- maltodextrine : 10 à 20 %.

### 1. Caractéristiques de la poudre imprégnée selon l'invention

Ecoulement : 7,17 sec
Masse volumique apparente : 0,67 g/cm³
Masse volumique tassée : 0,78 g/cm³
Analyse granulométrique par tamisage (Retsch) :

**[Tableau 1]**

| Tamis (µm) | Refus par tamis (%) |
|---|---|
| 0 | 3,2 |
| 25 | 17,1 |
| 50 | 17,1 |
| 100 | 32,6 |
| 200 | 19.0 |
| 315 | 10,4 |
| 500 | 0,3 |
| 710 | 0,3 |
| 1000 | 0,00 |

### 2. Caractéristiques d'une poudre imprégnée de l'art antérieur

Ecoulement : 9,42 sec (avec une impulsion au début de chaque écoulement) - Poudre électrostatique
Masse volumique apparente Densité apparente : 0,61 g/cm³
Masse volumique tassée : 0,70 g/cm³
Analyse granulométrique par tamisage (Retsch) :

**[Tableau 2]**

| Tamis (µm) | Refus par tamis (%) |
|---|---|
| 0 | 1,9 |
| 25 | 6,6 |
| 50 | 18,1 |
| 100 | 40,2 |
| 200 | 21,6 |
| 315 | 7,9 |
| 500 | 1,8 |
| 710 | 1,9 |
| 1000 | 0,1 |

### 3. Caractéristiques de l'extrait sec

Ecoulement : INFINI
Masse volumique apparente : 0,36 g/cm³
Masse volumique tassée : 0,5 g/cm³
Analyse granulométrique par tamisage (Retsch) :

**[Tableau 3]**

| Tamis (µm) | Refus par tamis (%) |
|---|---|
| 0 | 4,9 |
| 25 | 52,5 |
| 50 | 23,9 |
| 100 | 14,0 |
| 200 | 2,7 |
| 315 | 1,3 |
| 500 | 0,4 |
| 710 | 0,3 |
| 1000 | 0,3 |

### 4. Formulations de comprimés

Trois formulations de comprimés ont été étudiées :
- formulation à base d'une poudre imprégnée selon l'invention (A),
- formulation à base d'une poudre imprégnée selon l'art antérieur (B),
- extrait sec (C).

**[Tableau 4]**

| | A | B | C |
|---|---|---|---|
| Uniformité de masse | 908,5 mg | 905,6 mg | 898,6 mg |
| Epaisseur | 5,99 mm | 5,99 mm | 6,03 mm |
| Dureté | 207,5 N | 207,6 N | 262,2 N |
| Friabilité | 0,11% (4 min) | 0,15% (4 min) | 0,09% (4 min) |
| Résistance à la traction | 1,078 Mpa | 1,078 Mpa | 1,348 Mpa |
| Temps de désagrégation | 1 h 12 min 39 sec | 1 h 10 min 34 sec | 1 h 14 min 03 sec |

### 5. Comparaison des profils de dissolution

Une étude de dissolution a été réalisée sur les 3 formulations.

Les profils cumulés moyens de dissolution et les vitesses instantanées moyennes de dissolution sont comparées.

La méthode mise en œuvre est adaptée de la méthode de la Pharmacopée Européenne, monographie « 5.17.1 : Recommandations relatives à l'essai de dissolution» et « 4.1.3 : Solution Tampon phosphate pH 2,0 et solution tampon phosphate pour ajuster le pH à 6.8 ».

Les conditions générales sont les suivantes :
- milieu de dissolution : Solution tampon phosphate pH 2,0+/- 0,5 puis changement de pH après le prélèvement T1H à pH 6,8+/-0,5 avec le solution tampon phosphate ;
- Température du bain : 37°C,
- Vitesse d'agitation : 90 tours par minutes
- Volume de prélèvement : 1 ml,
- Nombre de prélèvements : T30 min, T1H, T1H30, T2H, T3H, T4H, T5H, T6H.

Les profils de dissolution cumulés du principe actif sont différents en fonction de la formulation testée pour un comprimé ayant une dureté similaire.

La libération du principe actif avec l'extrait sec atteint un palier dès 3 heures.

La granulométrie de la poudre imprégnée a un impact sur la libération du principe actif au cours du temps dans le milieu de dissolution, avec un profil de libération lente et progressive pour la poudre imprégnée selon l'invention.

## Revendications

1. Procédé de fabrication d'une poudre imprégnée comprenant un support biologique sur lequel est déposé un extrait biologique, lequel procédé de fabrication comprend :
- une étape de fourniture d'un extrait biologique concentré,
ledit extrait biologique concentré étant issu d'une opération d'extraction à partir d'une matière biologique suivi d'une opération de concentration dudit extrait biologique,
- une étape d'imprégnation dudit extrait biologique concentré sur ledit support biologique, et
- une étape de séchage dudit support biologique imprégné par ledit extrait biologique concentré, pour obtenir ladite poudre imprégnée,
**caractérisé en ce que** ledit procédé de fabrication comprend une étape de micronisation dudit support biologique et/ou de ladite poudre imprégnée, dont les paramètres sont ajustés pour obtenir une poudre imprégnée présentant, après ladite étape de séchage, les caractéristiques granulométriques suivantes :
- une taille inférieure, ou égale, à 50 µm représentant au maximum 25%, en poids,
- une taille inférieure, ou égale, à 100 µm représentant au maximum 50%, en poids, et
- une taille inférieure, ou égale, à 500µm représentant au minimum 90%, en poids.

2. Procédé de fabrication selon la revendication 1, **caractérisé en ce que** ladite poudre imprégnée présente :
- une masse volumique apparente allant de 0,3 à 0,8 g/cm³, et
- une masse volumique tassée allant de 0,4 à 0,9 g/cm³.

3. Procédé de fabrication selon l'une quelconque des revendications 1 ou 2, **caractérisé en ce que** :
- le support biologique est un support végétal, et
- l'extrait biologique est un extrait végétal.

4. Procédé de fabrication selon l'une quelconque des revendications 1 à 3, **caractérisé en ce que** ladite étape de micronisation est mise en œuvre sur le support biologique :
- préalablement à ladite étape d'imprégnation, et/ou
- entre ladite étape d'imprégnation et ladite étape de séchage, et/ou
- postérieurement à ladite étape de séchage.

5. Procédé de fabrication selon l'une quelconque des revendications 1 à 4, **caractérisé en ce que** ledit extrait biologique concentré est issu d'une opération d'extraction à partir d'une matière végétale fraîche ou sèche,
laquelle opération d'extraction consiste par exemple en une opération d'extraction aqueuse, éventuellement par un solvant consistant en un mélange hydro-alcoolique, avantageusement hydro-éthanolique, et encore plus avantageusement un mélange 50% / 50% en volume.

6. Procédé de fabrication selon l'une quelconque des revendications 1 à 5, **caractérisé en ce que** ledit extrait biologique concentré est issu d'une opération d'extraction réalisée sous chauffage, avantageusement sous hyperfréquence, et sous pression réduite ou est réalisée sous gaz inerte, avantageusement sous azote.

7. Procédé de fabrication selon l'une quelconque des revendications 1 à 6, **caractérisé en ce que** ledit extrait biologique concentré est issu d'une opération d'extraction mise à œuvre à une température inférieure ou égale à 95°C à 150 mbar, et encore plus avantageusement à une température de 55°C sous pression réduite de 150 mbar et **en ce qu'**elle a une durée inférieure à 1 heure, encore plus avantageusement de l'ordre de 15 minutes.

8. Procédé de fabrication selon l'une quelconque des revendications 1 à 7, **caractérisé en ce que** ledit extrait biologique concentré est issu d'une opération d'extraction mise à œuvre plusieurs fois, avantageusement trois fois,
et de préférence, entre chaque répétition, de la matière végétale est ajouté à l'extrait et/ou la température d'extraction est augmentée.

9. Procédé de fabrication selon l'une quelconque des revendications 1 à 8, **caractérisé en ce que** l'étape d'imprégnation dudit extrait biologique concentré sur ledit support biologique consiste en une étape de pulvérisation.

10. Procédé de fabrication selon l'une quelconque des revendications 1 à 9, **caractérisé en ce que** les paramètres de ladite étape de micronisation dudit support biologique et/ou de ladite poudre imprégnée sont ajustés pour obtenir une poudre imprégnée présentant, après ladite étape de séchage, les caractéristiques granulométriques suivantes :
- une taille inférieure, ou égale, à 50 µm représentant de 10 à 25% en poids,
- une taille inférieure, ou égale, à 100 µm représentant de préférence de 25 à 50% en poids, et
- une taille inférieure, ou égale, à 500µm représentant de préférence de 95 à 100% en poids.

11. Poudre imprégnée issue d'un procédé de fabrication selon l'une quelconque des revendications 1 à 10, comprenant un support biologique sous forme d'une poudre sur lequel est déposé un extrait biologique concentré,
**caractérisé en ce que** ladite poudre imprégnée présente les caractéristiques granulométriques suivantes :
- une taille inférieure, ou égale, à 50 µm représentant au maximum 25%, en poids, de préférence de 10 à 25%,
- une taille inférieure, ou égale, à 100 µm représentant au maximum 50%, en poids, de préférence de 25 à 50%, et
- une taille inférieure, ou égale, à 500µm représentant au minimum 90%, en poids, de préférence de 95 à 100%,
laquelle poudre imprégnée présente avantageusement :
- une masse volumique apparente, ou plus précisément une masse volumique apparente sans tassement, allant de 0,3 à 0,8 g/cm³, et
- une masse volumique tassée, ou masse volumique apparente après tassement, allant de 0,4 à 0,9 g/cm³.

12. Composition, avantageusement sous forme d'un comprimé, comprenant une poudre imprégnée selon la revendication 11 mélangée avec au moins un principe actif.
